# EUROPEAN PATENT SPECIFICATION

(11) **EP 3 603 695 B1**
(45) Date of publication and mention of the grant of the patent: **07.04.2021**
(21) Application number: 18774412.3
(22) Date of filing: 21.02.2018
(51) Int. Cl.: A61M 1/36

(54) **BLOOD PURIFICATION DEVICE AND PRIMING METHOD THEREOF**
BLUTREINIGUNGSVORRICHTUNG UND PRIMING-VERFAHREN DAFÜR
DISPOSITIF DE PURIFICATION DU SANG ET SON PROCÉDÉ D'AMORÇAGE

(30) Priority: 31.03.2017 JP 2017072430
(43) Date of publication of application: 05.02.2020
(73) Proprietor: Asahi Kasei Medical Co., Ltd., Tokyo 100-0006 (JP)
(72) Inventor: GOTANDA, Yuya, Kawagoe-shi Saitama 350-0833 (JP)
(74) Representative: dompatent von Kreisler Selting Werner - Partnerschaft von Patent- und Rechtsanwälten mbB
(86) International application number: PCT/JP2018/006250
(87) International publication number: WO 2018/180044

(56) References cited:
- JP-A- 2004 357 957
- JP-A- 2012 139 405
- JP-Y2- H0 519 076
- US-A1- 2003 135 152
- US-A1- 2011 213 289
- US-A1- 2014 217 020

## Description

### Technical Field

The present invention relates to a blood purification device and a priming method thereof.

### Background Art

Blood purification therapies have conventionally been performed by taking out the blood from a patient's body, removing a disease-causing substance, a specific leukocyte, or the like from this blood and reinfusing the resulting blood to the body. In such blood purification therapies, a blood purification device has been used which has a blood circuit for circulating the blood taken out from a body and reinfusing it thereto and a blood purifier equipped with a treating material such as adsorbing material or separating material for removing or separating a specific substance from the blood.

Before a blood purification treatment with such a blood purification device, it is the common practice to carry out "priming" which is a treatment of washing the inside of a blood circuit or blood treating unit to be used with a clean liquid (washing liquid) such as physiological saline or dialysate. Various techniques for automatically performing such priming have been proposed recently (refer to, for example, Patent Documents 1 and 2).

US2011/0213289 discloses a blood purification apparatus and a priming method for it, in particular, if during priming a bubble is detected, the priming solution is reintroduced in the system.

US2003/0135152 discloses a disposable cartridge for a blood perfusion system. It is disclosed that, in the case of bubble detection, a vent ventilates the air bubble.

US2014/0217020 discloses a modular fluid therapy system having jumpered flow paths and systems and methods for cleaning and disinfection. It is disclosed that once the priming mode is achieved, a degasing step begins by returning the flushing water to the reservoir.

### Citation List

### Patent Document

Patent Document 1: Japanese Patent Application Laid-Open No. 2005-253555
Patent Document 2: Japanese Patent Application Laid-Open No. 2007-190068

### Summary

### Technical Problem

Due to consumption of the washing liquid during priming, a washing liquid reservoir for storing the washing liquid therein sometimes becomes empty. In order to prevent such a situation, air bubbles have conventionally been removed by providing an empty detector in a washing flow path connected to the washing liquid reservoir, automatically stopping priming when this empty detector detects air bubbles, and subsequently filling the washing flow path between the drip chamber and the washing liquid reservoir with the washing liquid by crushing a drip chamber provided in the washing flow path by a user.

Since the operation of crushing a drip chamber is cumbersome, however, there has recently been investigated a technique of performing, when the empty detector detects air bubbles, reverse rotation of a pump provided in the washing flow path, thereby automatically removing air bubbles. Reverse rotation of the pump is however accompanied with the drawback that it disturbs maintenance of cleanliness because a waste liquid after washing or air is sucked in from the other end of the blood circuit.

With the foregoing in view, the present invention has been made. The purpose is to, when air bubbles are detected during priming of a blood purification device, automatically remove the air bubbles while keeping cleanliness of the device.

### Solution to Problem

In order to achieve the above-described purpose, a blood purification device of the present invention comprises: a blood purifier; a washing liquid reservoir having a washing liquid stored therein; a washing flow path that connects the blood purifier and the washing liquid reservoir to each other, a detector provided on the washing flow path for detecting an air bubble; a discharge flow path communicated with the blood purifier for discharging the washing liquid; a transfusion reservoir for storing a transfusion therein; a pump provided on the flow path between the detector and the transfusion reservoir; and a control unit that activates the pump to supply the transfusion from the transfusion reservoir to the washing liquid reservoir via the detector when the detector detects an air bubble.

A priming method of the present invention is a priming method of a blood purification device equipped with: a blood purifier; a washing liquid reservoir having a washing liquid stored therein, a washing flow path that connects the blood purifier and the washing liquid reservoir to each other; a detector provided on the washing flow path for detecting an air bubble; a discharge flow path communicated with the blood purifier for discharging the washing liquid; a transfusion reservoir for storing a transfusion therein; and a pump provided on the flow path between the detector and the transfusion reservoir. The method includes a step of activating the pump so as to supply the transfusion from the transfusion reservoir to the washing liquid reservoir via the detector when the detector detects an air bubble during priming.

By using such a configuration and method, when the detector provided on the washing flow path detects air bubbles, the pump can be activated so as to supply the transfusion from the transfusion reservoir to the washing liquid reservoir via the detector. The air bubbles present in the washing flow path can therefore be removed by the aid of the transfusion. Only by reverse rotation of the pump placed on the washing flow path or discharge flow path for removing air bubbles present in the washing flow path, the used waste liquid or air is inevitably sucked in from the end of the discharge flow path. By activating the pump provided between the detector and the transfusion reservoir and supplying the transfusion from the transfusion reservoir to the washing liquid reservoir via the detector as in the present device, it is possible to prevent the used waste liquid or air from being sucked in from the end of the discharge flow path and to keep cleanliness of the device.

The blood purification device of the present invention can be equipped with a transfusion flow path that connects the transfusion reservoir and the discharge flow path to each other, a first opening/closing valve for opening or closing the transfusion flow path, and a second opening/closing valve provided in the discharge flow path on a side downstream of the transfusion flow path for opening or closing the discharge flow path and can be provided with a pump on the discharge flow path between the detector and the transfusion flow path. In such a case, at the time of priming, the control unit can close the transfusion flow path by means of the first opening/closing valve, open the second opening/closing valve, and cause forward rotation of the pump; and when the detector detects air bubbles, the control unit can supply the transfusion from the transfusion reservoir to the washing liquid reservoir via the detector by opening the transfusion flow path by means of the first opening/closing valve, closing the second opening/closing valve, and causing reverse rotation of the pump (first embodiment).

The blood purification device of the present invention can comprise a transfusion flow path that connects the transfusion reservoir and the discharge flow path to each other and a priming pump provided on the discharge flow path on a side downstream of the transfusion flow path and can use, as the pump, a transfusion pump provided on the transfusion flow path. In such a case, at the time of priming, the control unit can cause forward rotation of the priming pump; and when the detector detects air bubbles, the control unit can supply the transfusion from the transfusion reservoir to the washing liquid reservoir via the detector by stopping the priming pump and activating the transfusion pump (second embodiment).

The blood purification device of the present invention can comprise a transfusion flow path that connects the transfusion reservoir and the discharge flow path to each other and a transfusion pump provided on the transfusion flow path and can use, as the pump, a priming pump provided on the discharge flow path on a side upstream of the transfusion flow path. In such a case, at the time of priming, the control unit can cause forward operation of the priming pump; and when the detector detects air bubbles, the control unit can supply the transfusion from the transfusion reservoir to the washing liquid reservoir via the detector by causing reverse rotation of the priming pump and activating the transfusion pump. In causing reverse rotation of the priming pump and activating the transfusion pump, the control unit can control the flow rate of the priming pump to be smaller than the flow rate of the transfusion pump in (third embodiment).

The blood purification device of the present invention can be equipped with a transfusion flow path that connects the transfusion reservoir and the washing flow path or blood purifier to each other and a priming pump provided on the discharge flow path and can use, as the pump, a transfusion pump provided on the transfusion flow path. In such a case, at the time of priming, the control unit can cause forward rotation of the priming pump; and when the detector detects air bubbles, the control unit can supply the transfusion from the transfusion reservoir to the washing liquid reservoir via the detector by stopping the priming pump and activating the transfusion pump (fourth embodiment).

The blood purification devices according to the first to fourth embodiments can use, as the washing flow path, a venous line that connects a patient's blood return part and the blood purifier to each other.

The blood purification device of the present invention can be equipped with a transfusion flow path that connects the transfusion reservoir and the discharge flow path to each other, a first opening/closing valve for opening or closing the transfusion flow path, and a second opening/closing valve provided on the discharge flow path on a side downstream of the transfusion flow path, and can have a pump on the washing flow path. In such a case, at the time of priming, the control unit can close the transfusion flow path by means of the first opening/closing valve, open the discharge flow path by means of the second opening/closing valve, and cause forward rotation of the pump; and when the detector detects air bubbles, the control unit can supply the transfusion from the transfusion reservoir to the washing liquid reservoir via the detector by opening the transfusion flow path by means of the first opening/closing valve, closing the discharge flow path by means of the second opening/closing valve, and causing reverse rotation of the pump (fifth embodiment).

The blood purification device of the present invention can be equipped with a transfusion flow path that connects the transfusion reservoir and the discharge flow path to each other and a priming pump provided on the washing flow path and can use, as the pump, a transfusion pump provided on the transfusion flow path. In such a case, at the time of priming, the control unit can cause forward rotation of the priming pump; and when the detector detects air bubbles, the control unit can supply the transfusion from the transfusion reservoir to the washing liquid reservoir via the detector by causing reverse rotation of the priming pump and activating the transfusion pump (sixth embodiment).

The blood purification device of the present invention can be equipped with a transfusion flow path that connects the transfusion reservoir and the washing flow path to each other and a priming pump provided on the washing flow path on a side upstream of the transfusion flow path, and can use, as the pump, a transfusion pump provided on the transfusion flow path. In such a case, at the time of priming, the control unit can cause forward rotation of the priming pump; and when the detector detects air bubbles, the control unit can supply the transfusion from the transfusion reservoir to the washing liquid reservoir via the detector by causing reverse rotation of the priming pump and activating the transfusion pump (seventh embodiment).

The blood purification device of the present invention can be equipped with a transfusion flow path that connects the transfusion reservoir and the blood purifier to each other and a priming pump provided on the washing flow path and can use, as the pump, a transfusion pump provided on the transfusion flow path. In such a case, at the time of priming, the control unit can cause forward rotation of the priming pump; and when the detector detects air bubbles, the control unit can supply the transfusion from the transfusion reservoir to the washing liquid reservoir via the detector by causing reverse rotation of the priming pump and activating the transfusion pump (eighth embodiment).

In the blood purification devices according to the sixth to eighth embodiments, the control unit can control the flow rate of the priming pump to be smaller than the flow rate of the transfusion pump in causing reverse rotation of the priming pump and activating the transfusion pump.

The blood purification device of the present invention can be equipped with a transfusion flow path that connects the transfusion reservoir and the washing flow path to each other and a priming pump provided on the washing flow path on a side downstream of the transfusion flow path, and can use, as the pump, a transfusion pump provided on the transfusion flow path. In such a case, at the time of priming, the control unit can cause forward rotation of the priming pump; and when the detector detects air bubbles, the control unit can supply the transfusion from the transfusion reservoir to the washing liquid reservoir via the detector by stopping the priming pump and activating the transfusion pump (ninth embodiment).

The blood purification devices according to the fifth to ninth embodiments can use, as the washing flow path, an arterial line that connects a patient's blood removal part and the blood purifier to each other. In addition, in the blood purification devices according to the fifth to ninth embodiments, the washing flow path may have a priming line placed in the arterial line that connects a patient's blood removal part and the blood purifier to each other.

In the blood purification devices of the present invention, when the detector detects air bubbles, the control unit can give an alarm after activating the pump in a predetermined manner. In addition, when the detector detects air bubbles, the control unit can give an alarm, stop the pump, and after the alarm is cleared, activate the pump.

### Advantageous Effects of Invention

According to the present invention, when air bubbles are detected during priming of a blood purification device, the air bubbles can be removed automatically while keeping cleanliness of the device.

### Brief Description of Drawings

Fig. 1 is a block diagram for describing the constitution of a blood purification device according to a first embodiment of the present invention.
Fig. 2 is a block diagram for describing the constitution of a blood purification device according to a second embodiment of the present invention.
Fig. 3 is a block diagram for describing the constitution of a blood purification device according to a third embodiment of the present invention.
Fig. 4 is a block diagram for describing the constitution of a blood purification device according to a fourth embodiment of the present invention.
Fig. 5 is a block diagram for describing a modification example of the blood purification device according to the fourth embodiment of the present invention.
Fig. 6 is a block diagram for describing the constitution of a blood purification device according to a fifth embodiment of the present invention.
Fig. 7 is a block diagram for describing the constitution of a blood purification device according to a sixth embodiment of the present invention.
Fig. 8 is a block diagram for describing the constitution of a blood purification device according to a seventh embodiment of the present invention.
Fig. 9 is a block diagram for describing the constitution of a blood purification device according to an eighth embodiment of the present invention.
Fig. 10 is a block diagram for describing the constitution of a blood purification device according to a ninth embodiment of the present invention.
Fig. 11 is a block diagram for describing a modification example of the blood purification devices according to the fifth to ninth embodiments of the present invention.

### Description of Embodiments

Embodiments of the present invention will hereinafter be described referring to the drawings. The following embodiments are only preferable application examples and do not limit the application range of the present invention.

### <First Embodiment

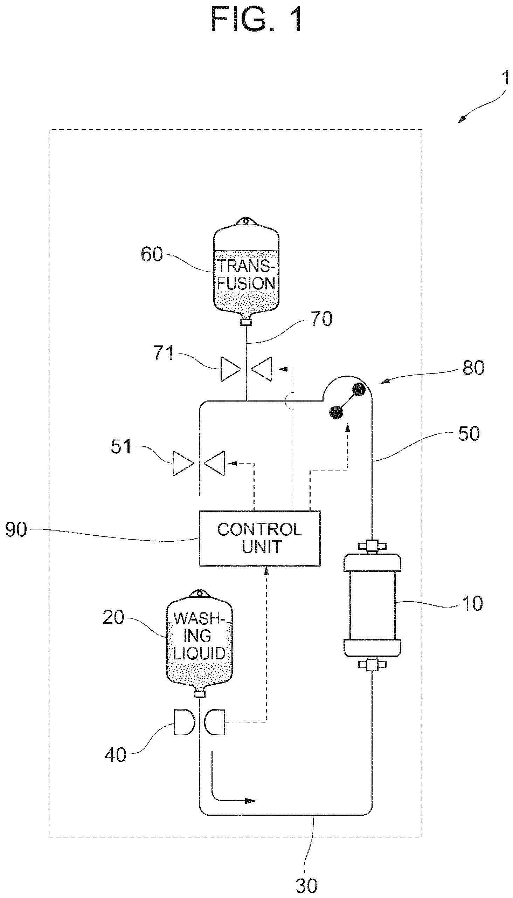

First, the constitution of a blood purification device 1 according to the first embodiment of the present invention is described referring to Fig. 1

The blood purification device 1 according to the present embodiment is used for so-called leukocyte apheresis (LCAP) and as shown in Fig. 1, it is equipped with a blood purifier 10, a washing liquid reservoir 20, a washing flow path 30, an air bubble detector 40, a discharge flow path 50, a transfusion reservoir 60, a transfusion flow path 70, a priming pump 80, and a control unit 90.

The blood purifier 10 purifies the blood introduced from a blood inlet by removing an activated leukocyte contained in the blood by a filter and discharges the purified blood from a blood outlet. As the filter, that having an adsorbing material for removing a leukocyte can be used.

The washing liquid reservoir 20 is a vessel for storing a washing liquid for priming (for example, physiological saline) and is connected to an end of the washing flow path 30. The washing flow path 30 is a flow path that connects the blood purifier 10 and the washing liquid reservoir 20 to each other to deliver a washing liquid supplied from the washing liquid reservoir 20 to the blood purifier 10. The present embodiment uses, as the washing flow path 30, a venous line that connects a patient's blood return part and the blood purifier 10 to each other.

The air bubble detector 40 is provided on the washing flow path 30 for detecting air bubbles contained in a washing liquid delivered in the washing flow path 30. Information on the detection results of the air bubble detector 40 is sent to the control unit 90 and used for control of the priming pump 80.

The discharge flow path 50 is a flow path for delivering the washing liquid which has passed the blood purifier 10 and discharging the liquid to the outside and is communicated with the blood purifier 10. The present embodiment uses, as the discharge flow path 50, an arterial line that connects the patient's blood removal part and the blood purifier 10 to each other. The discharge flow path 50 has, on a side downstream of the transfusion flow path 70, a second opening/closing valve 51 which is activated under the control of the control unit 90 and opens or closes the discharge flow path 50.

The transfusion reservoir 60 is a vessel for storing a transfusion (for example, physiological saline) therein and is connected to the discharge flow path 50 via the transfusion flow path 70. The transfusion flow path 70 is provided with a first opening/closing valve 71 which is activated under the control of the control unit 90 and opens or closes the transfusion flow path 70.

The priming pump 80 is provided on the discharge flow path 50 on a side upstream of the transfusion flow path 70 (between the air bubble detector 40 and the transfusion reservoir 60) and achieves a priming treatment by forward rotation under the control of the control unit 90. The priming pump 80 also achieves an air bubble removal treatment by reverse rotation under the control of the control unit 90. In the present embodiment, the terms "on a side upstream" and "on a side downstream" are based on a flow direction of a washing liquid at the time of priming and "on the side of the washing liquid reservoir 20" and "on the side of the second opening/closing valve 51" are called "on a side upstream" and "on a side downstream", respectively..

The control unit 90 is comprised of, for example, a computer equipped with a memory, CPU (Central Processing Unit), and various programs loaded in the memory are performed by CPU to control various units of the blood purification device 1.

The control unit 90 in the present embodiment achieves a priming treatment (treatment of delivering a washing liquid in the washing liquid reservoir 20 to the blood purifier 10 via the washing flow path 30 and then discharging the liquid via the discharge flow path 50) by closing the transfusion flow path 70 by means of the first opening/closing valve 71, opening the discharge flow path 50 by means of the second opening/closing valve 51, and causing forward rotation of the priming pump 80. On the other hand, when the air bubble detector 40 detects air bubbles during priming, the control unit 90 achieves an air bubble removal treatment (treatment of supplying a transfusion in the transfusion reservoir 60 to the washing liquid reservoir 20 via the transfusion flow path 70, the discharge flow path 50, the blood purifier 10, and the washing flow path 30 in order of mention) by opening the transfusion flow path 70 by means of the first opening/closing valve 71, closing the discharge flow path 50 by means of the second opening/closing valve 51, and causing reverse rotation of the priming pump 80. The term "forward rotation" of the priming pump 80 means rotation of the priming pump 80 so as to deliver the washing liquid from the upstream side to the downstream side, while the term "reverse rotation" means rotation of the priming pump 80 in a direction opposite thereto.

When the air bubble detector 40 detects air bubbles during priming, the control unit 90 in the present embodiment gives an alarm after activating the priming pump 80 in a predetermined manner. When the air bubble detector 40 detects air bubbles during priming, the control unit 90 can also give an alarm and stop the priming pump 80, and after the alarm is cleared, activate the priming pump 80. The control unit 90 can also activate, based on the instruction of an operator, the priming pump 80 after the alarm is cleared.

Next, a priming method of the blood purification device 1 according to the present embodiment will be described.

The control unit 90 of the blood purification device 1 achieves a treatment (priming treatment) of delivering the washing liquid in the washing liquid reservoir 20 to the blood purifier 10 via the washing flow path 30 and then discharging the liquid via the discharge flow path 50 by closing the transfusion flow path 70 by means of the first opening/closing valve 71, opening the discharge flow path 50 by means of the second opening/closing valve 51, and causing forward rotation of the priming pump 80 (priming step).

Here, when the air bubble detector 40 detects air bubbles during priming, the control unit 90 achieves a treatment (air bubble removal treatment) of supplying the transfusion in the transfusion reservoir 60 to the washing liquid reservoir 20 via the transfusion flow path 70, the discharge flow path 50, the blood purifier 10, and the washing flow path 30 in order of mention by opening the transfusion flow path 70 by means of the first opening/closing valve 71, closing the discharge flow path 50 by means of the second opening/closing valve 51, and causing reverse rotation of the priming pump 80 (air bubble removal step).

When the air bubble detector 40 provided on the washing flow path 30 detects air bubbles during priming, the blood purification device 1 according to the embodiment described above can activate the priming pump 80 so as to supply the transfusion from the transfusion reservoir 60 to the washing liquid reservoir 20 via the air bubble detector 40. The air bubbles present in the washing flow path 30 can therefore be removed by the aid of the transfusion. Only by reverse rotation of the priming pump 80 placed in the discharge flow path 50 in order to remove air bubbles present in the washing flow path 30, the used waste liquid or air is inevitably sucked in from the end of the discharge flow path 50. By activating the priming pump 80 provided between the air bubble detector 40 and the transfusion reservoir 60 and supplying the transfusion from the transfusion reservoir 60 to the washing liquid reservoir 20 via the air bubble detector 40 as in the present device 1, on the other hand, it is possible to prevent the used waste liquid or air from being sucked in from the end of the discharge flow path 50 and to keep cleanliness of the device 1.

### <Second Embodiment

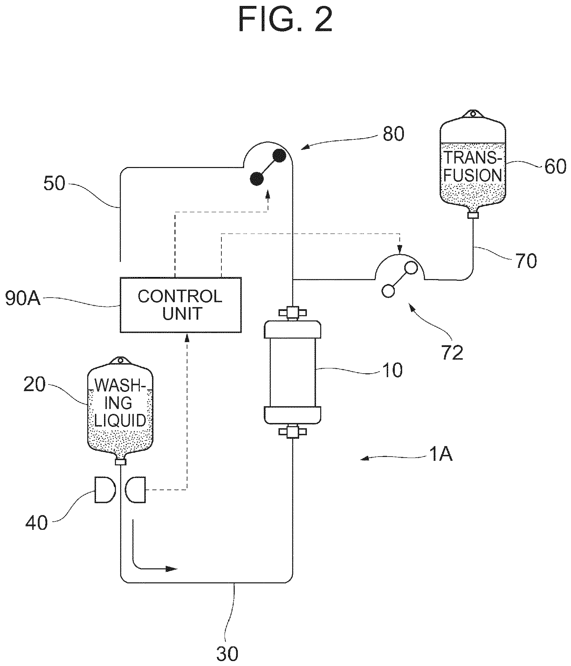

Next, a blood purification device 1A according to the second embodiment of the present invention will be described referring to Fig. 2. The blood purification device 1A according to the second embodiment has a constitution substantially similar to that of the first embodiment except that the control mode of the first embodiment is changed by omitting the opening/closing valves 51 and 71, changing the position of the transfusion flow path 70, and providing a transfusion pump 72. The components common to those of the first embodiment are denoted with the same reference signs as those in the first embodiment and a detailed description on these components is omitted.

The blood purification device 1A according to the present embodiment is equipped with, as shown in Fig. 2, a blood purifier 10, a washing liquid reservoir 20, a washing flow path 30, an air bubble detector 40, a discharge flow path 50, a transfusion reservoir 60, a transfusion flow path 70, a transfusion pump 72, a priming pump 80, and a control unit 90A. The blood purifier 10, the washing liquid reservoir 20, the washing flow path 30, the air bubble detector 40, the discharge flow path 50, the transfusion reservoir 60, and the priming pump 80 are similar to those described in the first embodiment so that a detailed description on them is omitted.

In the present embodiment, as shown in Fig. 2, the transfusion flow path 70 is provided on a side upstream of the priming pump 80 on the discharge flow path 50. This means that the priming pump 80 in the present embodiment is placed on the discharge flow path 50 on a side downstream of the transfusion flow path 70. In the present embodiment, the terms "on a side upstream" and "on a side downstream" are based on a flow direction of a washing liquid at the time of priming and "on the side of the washing liquid reservoir 20" and "on the end side of the discharge flow path 50" are called "on a side upstream" and "on a side downstream", respectively.

The transfusion pump 72 is provided on the transfusion flow path 70 as shown in Fig. 2 and is activated under the control of the control unit 90A to achieve the air bubble removal treatment.

The control unit 90A in the present embodiment achieves a priming treatment (treatment of delivering a washing liquid in the washing liquid reservoir 20 to the blood purifier 10 via the washing flow path 30 and then discharging the liquid via the discharge flow path 50) by causing forward rotation of the priming pump 80. On the other hand, when the air bubble detector 40 detects air bubbles during priming, the control unit 90A achieves an air bubble removal treatment (treatment of supplying a transfusion in the transfusion reservoir 60 to the washing liquid reservoir 20 via the transfusion flow path 70, the discharge flow path 50, the blood purifier 10, and the washing flow path 30 in order of mention) by stopping the priming pump 80 and activating the transfusion pump 72. The term "activating" the transfusion pump 72 means rotating the transfusion pump 72 so as to supply the transfusion from the transfusion reservoir 60 to the discharge flow path 50.

When the air bubble detector 40 detects air bubbles during priming, the control unit 90A in the present embodiment gives an alarm after activating the transfusion pump 72 in a predetermined manner. When the air bubble detector 40 detects air bubbles during priming, the control unit 90A can also give an alarm and stop the transfusion pump 72, and after the alarm is cleared, activate the transfusion pump 72. The control unit 90A can also activate, based on the instruction of an operator, the transfusion pump 72 after the alarm is cleared.

Next, a priming method of the blood purification device 1A according to the present embodiment will be described.

The control unit 90A of the blood purification device 1A achieves a treatment (priming treatment) of delivering the washing liquid in the washing liquid reservoir 20 to the blood purifier 10 via the washing flow path 30 and then discharging the liquid via the discharge flow path 50 by causing forward rotation of the priming pump 80 (priming step).

Here, when the air bubble detector 40 detects air bubbles during priming, the control unit 90A achieves a treatment (air bubble removal treatment) of supplying the transfusion in the transfusion reservoir 60 to the washing liquid reservoir 20 via the transfusion flow path 70, the discharge flow path 50, the blood purifier 10, and the washing flow path 30 in order of mention by stopping the priming pump 80 and activating the transfusion pump 72 (air bubble removal step).

The blood purification device 1A according to the embodiment described above can produce a working effect similar to that of the blood purification device 1 according to the first embodiment.

### <Third Embodiment

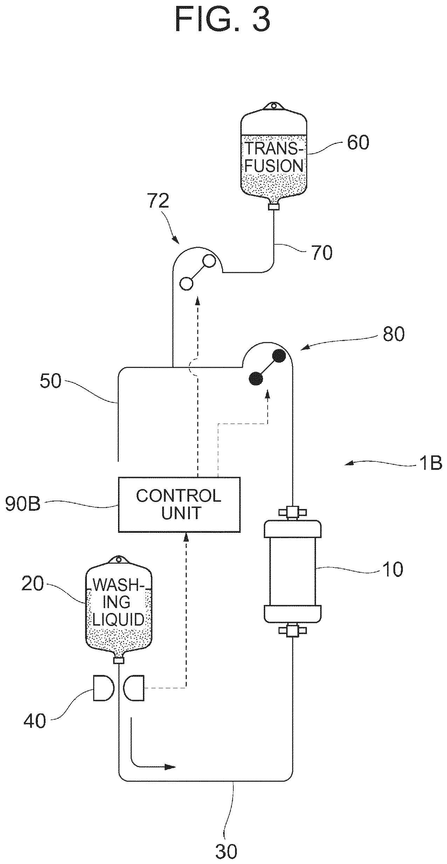

Next, a blood purification device 1B according to the third embodiment of the present invention will be described referring to Fig. 3. The blood purification device 1B according to the present embodiment has a constitution substantially similar to that of the second embodiment except that the position of the transfusion flow path 70 and the control mode in the second embodiment are changed. The components common to those of the second embodiment are denoted with the same reference signs as those in the second embodiment and a detailed description on them is omitted.

The blood purification device 1B according to the present embodiment is equipped with, as shown in Fig. 3, a blood purifier 10, a washing liquid reservoir 20, a washing flow path 30, an air bubble detector 40, a discharge flow path 50, a transfusion reservoir 60, a transfusion flow path 70, a transfusion pump 72, a priming pump 80, a control unit 90B, and the like. The blood purifier 10, the washing liquid reservoir 20, the washing flow path 30, the air bubble detector 40, the discharge flow path 50, the transfusion reservoir 60, the transfusion pump 72, and the priming pump 80 are similar to those of the second embodiment so that a detailed description on them is omitted.

In the present embodiment, as shown in Fig. 3, the transfusion flow path 70 is provided on a side downstream of the priming pump 80 on the discharge flow path 50. This means that the priming pump 80 in the present embodiment is placed on the discharge flow path 50 on a side upstream of the transfusion flow path 70. In the present embodiment, the terms "on a side upstream" and "on a side downstream" are based on a flow direction of a washing liquid at the time of priming and "on the side of the washing liquid reservoir 20" and "on the end side of the discharge flow path 50" are called "on a side upstream" and "on a side downstream", respectively.

The control unit 90B in the present embodiment achieves a priming treatment (treatment of delivering a washing liquid in the washing liquid reservoir 20 to the blood purifier 10 via the washing flow path 30 and then discharging the liquid via the discharge flow path 50) by causing forward rotation of the priming pump 80. On the other hand, when the air bubble detector 40 detects air bubbles during priming, the control unit 90B achieves an air bubble removal treatment (treatment of supplying a transfusion in the transfusion reservoir 60 to the washing liquid reservoir 20 via the transfusion flow path 70, the discharge flow path 50, the blood purifier 10, and the washing flow path 30 in order of mention) by causing reverse rotation of the priming pump 80 and activating the transfusion pump 72. In causing reverse rotation of the priming pump 80 and activating the transfusion pump 72, the control unit 90B controls the flow rate of the priming pump 80 to be equal to or smaller than the flow rate of the transfusion pump 72. The term "forward rotation" of the priming pump 80 means rotation of the priming pump 80 so as to deliver the washing liquid from the upstream side to the downstream side, while the term "reverse rotation" means rotation of the priming pump 80 in a direction opposite thereto. The term "activating" the transfusion pump 72 means rotating the transfusion pump 72 so as to supply the transfusion from the transfusion reservoir 60 to the discharge flow path 50.

When the air bubble detector 40 detects air bubbles during priming, the control unit 90B in the present embodiment gives an alarm after activating the priming pump 80 and the transfusion pump 72 in a predetermined manner. When the air bubble detector 40 detects air bubbles during priming, the control unit 90B can also give an alarm and stop the priming pump 80 and the transfusion pump 72, and after the alarm is cleared, activate the priming pump 80 and the transfusion pump 72. The control unit 90B can also activate, based on the instruction of an operator, the priming pump 80 and the transfusion pump 72 after the alarm is cleared.

Next, a priming method of the blood purification device 1 B according to the present embodiment will be described.

The control unit 90B of the blood purification device 1B achieves a treatment (priming treatment) of delivering the washing liquid in the washing liquid reservoir 20 to the blood purifier 10 via the washing flow path 30 and then discharging the liquid via the discharge flow path 50 by causing forward rotation of the priming pump 80 (priming step).

Here, when the air bubble detector 40 detects air bubbles during priming, the control unit 90B achieves a treatment (air bubble removal treatment) of supplying the transfusion in the transfusion reservoir 60 to the washing liquid reservoir 20 via the transfusion flow path 70, the discharge flow path 50, the blood purifier 10, and the washing flow path 30 in order of mention by causing reverse rotation of the priming pump 80 and activating the transfusion pump 72 (air bubble removal step).

The blood purification device 1B according to the embodiment described above can produce a working effect similar to that of the blood purification device 1 according to the first embodiment.

### <Fourth Embodiment

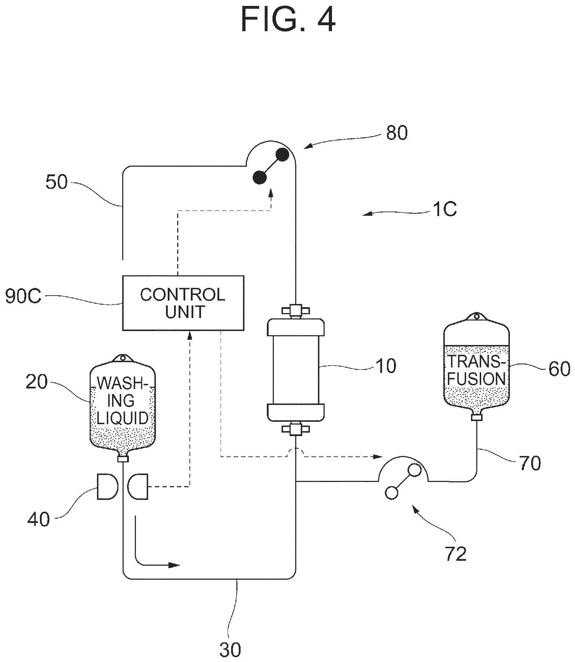

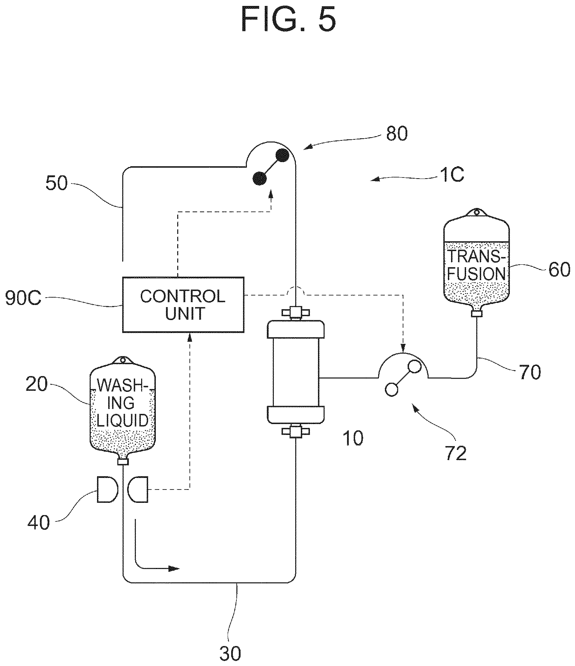

Next, a blood purification device 1C according to the fourth embodiment of the present invention will be described referring to Figs. 4 and 5. The blood purification device 1C according to the present embodiment has a constitution substantially similar to that of the second embodiment except that the position of the transfusion flow path 70 and also the control mode in the second embodiment are changed. The components common to those of the second embodiment are denoted with the same reference signs as those in the second embodiment and a detailed description on them is omitted.

The blood purification device 1C according to the present embodiment is equipped with, as shown in Fig. 4, a blood purifier 10, a washing liquid reservoir 20, a washing flow path 30, an air bubble detector 40, a discharge flow path 50, a transfusion reservoir 60, a transfusion flow path 70, a transfusion pump (infusion pump) 72, a priming pump 80, and a control unit 90C. The blood purifier 10, the washing liquid reservoir 20, the washing flow path 30, the air bubble detector 40, the discharge flow path 50, the transfusion reservoir 60, the transfusion pump 72, and the priming pump 80 are similar to those described in the second embodiment so that a detailed description on them is omitted.

In the present embodiment, as shown in Fig. 4, the transfusion flow path 70 is connected to the washing flow path 30. The control unit 90C in the present embodiment achieves a priming treatment (treatment of delivering a washing liquid in the washing liquid reservoir 20 to the blood purifier 10 via the washing flow path 30 and then discharging the liquid via the discharge flow path 50) by causing forward rotation of the priming pump 80. On the other hand, when the air bubble detector 40 detects air bubbles during priming, the control unit 90C achieves an air bubble removal treatment (treatment of supplying a transfusion in the transfusion reservoir 60 to the washing liquid reservoir 20 via the transfusion flow path 70 and the washing flow path 30 in order of mention) by stopping the priming pump 80 and activating the transfusion pump 72. The term "forward rotation" of the priming pump 80 means rotation of the priming pump 80 so as to deliver the washing liquid from the upstream side to the downstream side and the term "activating" the transfusion pump 72 means rotating the transfusion pump 72 so as to supply the transfusion from the transfusion reservoir 60 to the washing flow path 30.

When the air bubble detector 40 detects air bubbles during priming, the control unit 90C in the present embodiment gives an alarm after activating the transfusion pump 72 in a predetermined manner. When the air bubble detector 40 detects air bubbles during priming, the control unit 90C can also give an alarm and stop the transfusion pump 72, and after the alarm is cleared, activate the transfusion pump 72. The control unit 90C can also activate, based on the instruction of an operator, the transfusion pump 72 after the alarm is cleared.

Next, a priming method of the blood purification device 1C according to the present embodiment will be described.

The control unit 90C of the blood purification device 1C achieves a treatment (priming treatment) of delivering the washing liquid in the washing liquid reservoir 20 to the blood purifier 10 via the washing flow path 30 and then discharging the liquid via the discharge flow path 50 by causing forward rotation of the priming pump 80 (priming step).

Here, when the air bubble detector 40 detects air bubbles during priming, the control unit 90C achieves a treatment (air bubble removal treatment) of supplying the transfusion in the transfusion reservoir 60 to the washing liquid reservoir 20 via the transfusion flow path 70 and the washing flow path 30 in order of mention by stopping the priming pump 80 and activating the transfusion pump 72 (air bubble removal step).

The blood purification device 1C according to the embodiment described above can produce a working effect similar to that of the blood purification device 1 according to the first embodiment.

When the blood purifier 10 is a dialyzer or the like capable of delivering a liquid from the inside to the outside (or vice versa) of a membrane and at the same time having, on the outside of the purifier, a delivery port of a fluid such as dialysate, the transfusion flow path 70 may be connected to the blood purifier 10 as shown in Fig. 5 instead of connecting the transfusion flow path 70 to the washing flow path 30. Even if such a constitution is used, the control unit 90C can achieve the priming treatment and the air bubble removal treatment by performing a similar control. In such a case, the transfusion pump 72 functions as a dialysate pump.

### <Fifth Embodiment>

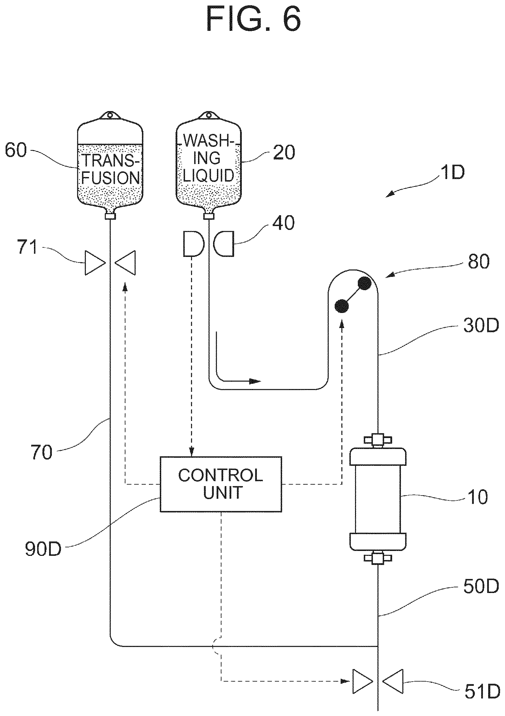

Next, a blood purification device 1D according to the fifth embodiment of the present invention will be described referring to Fig. 6. The blood purification device 1D according to the present embodiment is substantially similar to that of the first embodiment except that the respective positions of the washing flow path 30 and the discharge flow path 50 and also the control mode in the first embodiment are changed. The components common to those of the first embodiment are denoted with the same reference signs as those in the first embodiment and a detailed description on these components is omitted.

The blood purification device 1D according to the present embodiment is equipped with, as shown in Fig. 6, a blood purifier 10, a washing liquid reservoir 20, a washing flow path 30D, an air bubble detector 40, a discharge flow path 50D, a transfusion reservoir 60, a transfusion flow path 70, a priming pump 80, and a control unit 90D. The blood purifier 10, the washing liquid reservoir 20, the air bubble detector 40, the transfusion reservoir 60, the transfusion flow path 70, and the priming pump 80 are similar to those described in the first embodiment so that a detailed description on them is omitted.

In the present embodiment, an arterial line that connects a patient's blood removal part and the blood purifier 10 to each other is used as the washing flow path 30D and a venous line that connects a patient's blood return part and the blood purifier 10 to each other is used as the discharge flow path 50D. In the present embodiment, the priming pump 80 is provided on the washing flow path 30D and the transfusion flow path 70 is connected to the discharge flow path 50D.

The transfusion flow path 70 is provided with a first opening/closing valve 71 activated under the control of the control unit 90D for opening or closing the transfusion flow path 70. The discharge flow path 50D has, on a side downstream of the transfusion flow path 70, a second opening/closing valve 51D activated under the control of the control unit 90D for opening or closing the discharge flow path 50D. In the present embodiment, the term "on a side downstream" is based on a flow direction of a washing liquid at the time of priming and "on the side of the washing liquid reservoir 20" and "on the side of the second opening/closing valve 51D" are called "on a side upstream" and "on a side downstream", respectively. This means that the flow direction of the washing liquid at the time of priming in the present embodiment is opposite to the flow of the washing liquid in the first to fourth embodiments.

The control unit 90D in the present embodiment achieves a priming treatment (treatment of delivering a washing liquid in the washing liquid reservoir 20 to the blood purifier 10 via the washing flow path 30D and then discharging the liquid via the discharge flow path 50D) by closing the transfusion flow path 70 by means of the first opening/closing valve 71, opening the discharge flow path 50D by means of the second opening/closing valve 51D, and causing forward rotation of the priming pump 80. On the other hand, when the air bubble detector 40 detects air bubbles during priming, the control unit 90D achieves an air bubble removal treatment (treatment of supplying a transfusion in the transfusion reservoir 60 to the washing liquid reservoir 20 via the transfusion flow path 70, the discharge flow path 50D, the blood purifier 10, and the washing flow path 30D in order of mention) by opening the transfusion flow path 70 by means of the first opening/closing valve 71, closing the discharge flow path 50D by means of the second opening/closing valve 51D, and causing reverse rotation of the priming pump 80. The term "forward rotation" of the priming pump 80 means rotation of the priming pump 80 so as to deliver the washing liquid from the upstream side to the downstream side and the term "reverse rotation" means rotation of the priming pump 80 in a direction opposite thereto. The term "activating" the transfusion pump 72 means rotating the transfusion pump 72 so as to supply the transfusion from the transfusion reservoir 60 to the discharge flow path 50D.

When the air bubble detector 40 detects air bubbles during priming, the control unit 90D in the present embodiment gives an alarm after activating the priming pump 80 in a predetermined manner. When the air bubble detector 40 detects air bubbles during priming, the control unit 90D can also give an alarm and stop the priming pump 80, and after the alarm is cleared, activate the priming pump 80. The control unit 90D can also activate, based on the instruction of an operator, the priming pump 80 after the alarm is cleared.

Next, a priming method of the blood purification device 1D according to the present embodiment will be described.

The control unit 90D of the blood purification device 1D achieves a treatment (priming treatment) of delivering the washing liquid in the washing liquid reservoir 20 to the blood purifier 10 via the washing flow path 30D and then discharging the liquid via the discharge flow path 50D by causing forward rotation of the priming pump 80 (priming step).

Here, when the air bubble detector 40 detects air bubbles during priming, the control unit 90D achieves a treatment (air bubble removal treatment) of supplying the transfusion in the transfusion reservoir 60 to the washing liquid reservoir 20 via the transfusion flow path 70, the discharge flow path 50D, the blood purifier 10, and the washing flow path 30D in order of mention by opening the transfusion flow path 70 by means of the first opening/closing valve 71, closing the discharge flow path 50D by means of the second opening/closing valve 51D, and causing reverse rotation of the priming pump 80 (air bubble removal step).

The blood purification device 1D according to the embodiment described above can produce a working effect similar to that of the blood purification device 1 according to the first embodiment.

### <Sixth Embodiment

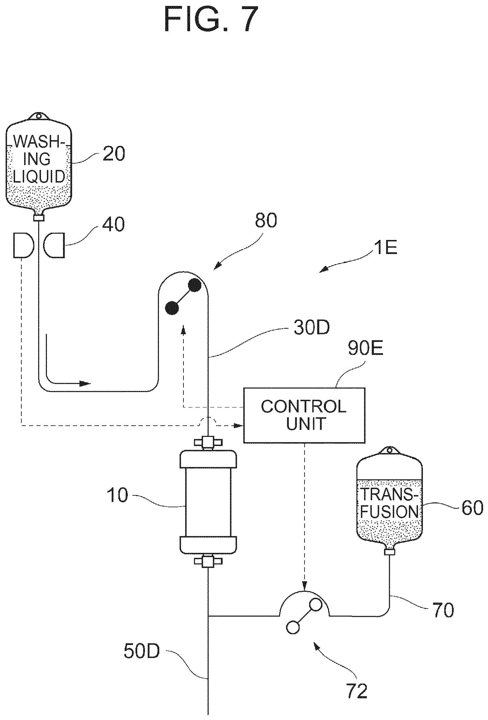

Next, a blood purification device 1E according to the sixth embodiment of the present invention will be described referring to Fig. 7. The blood purification device 1E according to the present embodiment is similar to that of the fifth embodiment except that the control mode is changed by providing a transfusion pump 72 while omitting the opening/closing valves 51D and 71 of the fifth embodiment. The components common to those of the fifth embodiment are denoted with the same reference signs as those in the fifth embodiment and a detailed description on these components is omitted.

The blood purification device 1E according to the present embodiment is equipped with, as shown in Fig. 7, a blood purifier 10, a washing liquid reservoir 20, a washing flow path 30D, an air bubble detector 40, a discharge flow path 50D, a transfusion reservoir 60, a transfusion flow path 70, a transfusion pump 72, a priming pump 80, and a control unit 90E. The blood purifier 10, the washing liquid reservoir 20, the washing flow path 30D, the air bubble detector 40, the discharge flow path 50D, the transfusion reservoir 60, the transfusion flow path 70, and the priming pump 80 are similar to those described in the fifth embodiment so that a detailed description on them is omitted.

The transfusion pump 72 in the present embodiment is provided on the transfusion flow path 70 as shown in Fig. 7 and achieves an air bubble removal treatment by being activated under the control of the control unit 90E. The control unit 90E in the present embodiment achieves a priming treatment (treatment of delivering a washing liquid in the washing liquid reservoir 20 to the blood purifier 10 via the washing flow path 30D and then discharging the liquid via the discharge flow path 50D) by causing forward rotation of the priming pump 80. On the other hand, when the air bubble detector 40 detects air bubbles during priming, the control unit 90E achieves an air bubble removal treatment (treatment of supplying a transfusion in the transfusion reservoir 60 to the washing liquid reservoir 20 via the transfusion flow path 70, the discharge flow path 50D, the blood purifier 10, and the washing flow path 30D in order of mention) by causing reverse rotation of the priming pump 80 and activating the transfusion pump 72. In causing reverse rotation of the priming pump 80 and activating the transfusion pump 72, the control unit 90E controls the flow rate of the priming pump 80 to be equal to or smaller than the flow rate of the transfusion pump 72. The term "forward rotation" of the priming pump 80 means rotation of the priming pump 80 so as to deliver the washing liquid from the upstream side to the downstream side and the term "reverse rotation" means rotation of the priming pump 80 in a direction opposite thereto. The term "activating" the transfusion pump 72 means rotating the transfusion pump 72 so as to supply the transfusion from the transfusion reservoir 60 to the discharge flow path 50D.

When the air bubble detector 40 detects air bubbles during priming, the control unit 90E in the present embodiment gives an alarm after activating the priming pump 80 and the transfusion pump 72 in a predetermined manner. When the air bubble detector 40 detects air bubbles during priming, the control unit 90E can also give an alarm and stop the priming pump 80 and the transfusion pump 72, and after the alarm is cleared, activate the priming pump 80 and the transfusion pump 72. The control unit 90E can also activate, based on the instruction of an operator, the priming pump 80 and the transfusion pump 72 after the alarm is cleared.

Next, a priming method of the blood purification device 1 E according to the present embodiment will be described.

The control unit 90E of the blood purification device 1E achieves a treatment (priming treatment) of delivering the washing liquid in the washing liquid reservoir 20 to the blood purifier 10 via the washing flow path 30D and then discharging the liquid via the discharge flow path 50D by causing forward rotation of the priming pump 80 (priming step).

Here, when the air bubble detector 40 detects air bubbles during priming, the control unit 90E achieves a treatment (air bubble removal treatment) of supplying the transfusion in the transfusion reservoir 60 to the washing liquid reservoir 20 via the transfusion flow path 70, the discharge flow path 50D, the blood purifier 10, and the washing flow path 30D in order of mention by causing reverse rotation of the priming pump 80 and activating the transfusion pump 72 (air bubble removal step).

The blood purification device 1E according to the embodiment described above can produce a working effect similar to that of the blood purification device 1 according to the first embodiment.

### <Seventh Embodiment

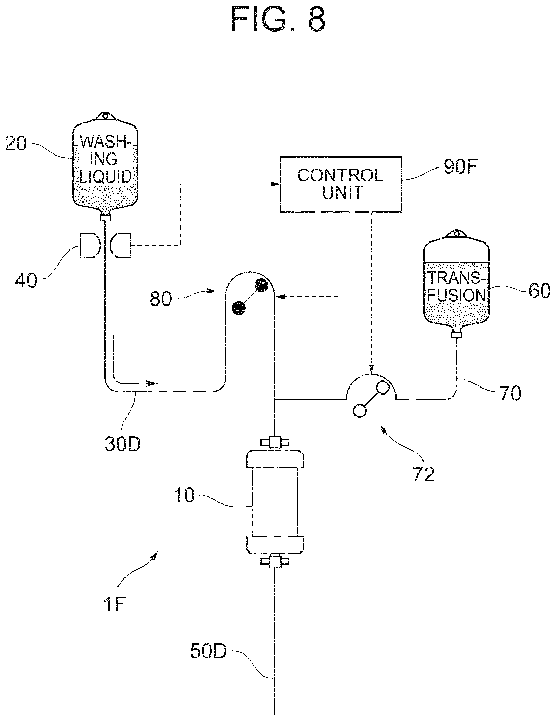

Next, a blood purification device 1F according to the seventh embodiment of the present invention will be described referring to Fig. 8. The blood purification device 1F according to the present embodiment has a constitution similar to that of the sixth embodiment except that the position of the transfusion flow path 70 and the control mode in the six embodiment are changed. The components common to those of the sixth embodiment are denoted with the same reference signs as those in the sixth embodiment and a detailed description on these components is omitted.

The blood purification device 1F according to the present embodiment is equipped with, as shown in Fig. 8, a blood purifier 10, a washing liquid reservoir 20, a washing flow path 30D, an air bubble detector 40, a discharge flow path 50D, a transfusion reservoir 60, a transfusion flow path 70, a transfusion pump 72, a priming pump 80, and a control unit 90F. The blood purifier 10, the washing liquid reservoir 20, the washing flow path 30D, the air bubble detector 40, the discharge flow path 50D, the transfusion reservoir 60, the transfusion pump 72, and the priming pump 80 are similar to those described in the sixth embodiment so that a detailed description on them is omitted.

In the present embodiment, as shown in Fig. 8, the transfusion flow path 70 is connected to the washing flow path 30D on a side downstream of the priming pump 80. This means that in the present embodiment, the priming pump 80 is placed on the washing flow path 30D on a side upstream of the transfusion flow path 70. In the present embodiment, the terms "on a side upstream" and "on a side downstream" are based on a flow direction of a washing liquid at the time of priming and "on the side of the washing liquid reservoir 20" and "on the end side of the discharge flow path 50D" are called "on a side upstream" and "on a side downstream", respectively.

The control unit 90F in the present embodiment achieves a priming treatment (treatment of delivering a washing liquid in the washing liquid reservoir 20 to the blood purifier 10 via the washing flow path 30D and then discharging the liquid via the discharge flow path 50D) by causing forward rotation of the priming pump 80. On the other hand, when the air bubble detector 40 detects air bubbles during priming, the control unit 90F achieves an air bubble removal treatment (treatment of supplying a transfusion in the transfusion reservoir 60 to the washing liquid reservoir 20 via the transfusion flow path 70 and the washing flow path 30D in order of mention) by causing reverse rotation of the priming pump 80 and activating the transfusion pump 72. In causing reverse rotation of the priming pump 80 and activating the transfusion pump 72, the control unit 90F controls the flow rate of the priming pump 80 to be equal to or smaller than the flow rate of the transfusion pump 72. The term "forward rotation" of the priming pump 80 means rotation of the priming pump 80 so as to deliver the washing liquid from the upstream side to the downstream side, while the term "reverse rotation" means rotation of the priming pump 80 in a direction opposite thereto. The term "activating" the transfusion pump 72 means rotating the transfusion pump 72 so as to supply the transfusion from the transfusion reservoir 60 to the washing flow path 30D.

When the air bubble detector 40 detects air bubbles during priming, the control unit 90F in the present embodiment gives an alarm after activating the priming pump 80 and the transfusion pump 72 in a predetermined manner. When the air bubble detector 40 detects air bubbles during priming, the control unit 90F can also give an alarm and stop the priming pump 80 and the transfusion pump 72, and after the alarm is cleared, activate the priming pump 80 and the transfusion pump 72. The control unit 90F can also activate, based on the instruction of an operator, the priming pump 80 and the transfusion pump 72 after the alarm is cleared.

Next, a priming method of the blood purification device 1F according to the present embodiment will be described.

The control unit 90F of the blood purification device 1F achieves a treatment (priming treatment) of delivering the washing liquid in the washing liquid reservoir 20 to the blood purifier 10 via the washing flow path 30D and then, discharging the liquid via the discharge flow path 50D by causing forward rotation of the priming pump 80 (priming step).

Here, when the air bubble detector 40 detects air bubbles during priming, the control unit 90F achieves a treatment (air bubble removal treatment) of supplying the transfusion in the transfusion reservoir 60 to the washing liquid reservoir 20 via the transfusion flow path 70 and the washing flow path 30D in order of mention by causing reverse rotation of the priming pump 80 and activating the transfusion pump 72 (air bubble removal step).

The blood purification device 1F according to the embodiment described above can produce a working effect similar to that of the blood purification device 1 according to the first embodiment.

### <Eighth Embodiment

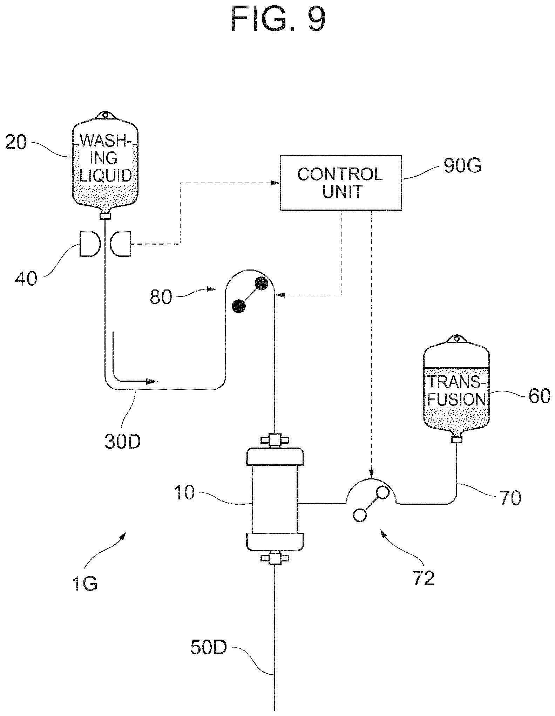

Next, a blood purification device 1G according to the eighth embodiment of the present invention will be described referring to Fig. 9. The blood purification device 1G according to the present embodiment has a constitution similar to that of the seventh embodiment except that the position of the transfusion flow path 70 in the seventh embodiment is changed. The components common to those of the seventh embodiment are denoted with the same reference signs as those in the seventh embodiment and a detailed description on these components is omitted. In the present embodiment, a dialyzer or the like capable of delivering a liquid from the inside to the outside (or vice versa) of a membrane and having, on the outside of the purifier, a delivery port of a fluid such as dialysate is used as the blood purifier 10.

In the blood purification device 1G according to the present embodiment, the transfusion flow path 70 is connected to the blood purifier 10 as shown in Fig. 9. The control unit 90G in the present embodiment achieves a priming treatment (treatment of delivering a washing liquid in the washing liquid reservoir 20 to the blood purifier 10 via the washing flow path 30D and then discharging the liquid via the discharge flow path 50D) by causing forward rotation of the priming pump 80. On the other hand, when the air bubble detector 40 detects air bubbles during priming, the control unit 90G achieves an air bubble removal treatment (treatment of supplying a transfusion in the transfusion reservoir 60 to the washing liquid reservoir 20 via the transfusion flow path 70, the blood purifier 10, and the washing flow path 30D in order of mention) by causing reverse rotation of the priming pump 80 and activating the transfusion pump 72. In causing reverse rotation of the priming pump 80 and activating the transfusion pump 72, the control unit 90G controls the flow rate of the priming pump 80 to be equal to or smaller than the flow rate of the transfusion pump 72.

When the air bubble detector 40 detects air bubbles during priming, the control unit 90G in the present embodiment gives an alarm after activating the priming pump 80 and the transfusion pump 72 in a predetermined manner. When the air bubble detector 40 detects air bubbles during priming, the control unit 90G can also give an alarm and stop the priming pump 80 and the transfusion pump 72, and after the alarm is cleared, activate the priming pump 80 and the transfusion pump 72. The control unit 90G can also activate, based on the instruction of an operator, the priming pump 80 and the transfusion pump 72 after the alarm is cleared.

Next, a priming method of the blood purification device 1G according to the present embodiment will be described.

The control unit 90G of the blood purification device 1G achieves a treatment (priming treatment) of delivering the washing liquid in the washing liquid reservoir 20 to the blood purifier 10 via the washing flow path 30D and then, discharging the liquid via the discharge flow path 50D by causing forward rotation of the priming pump 80 (priming step).

Here, when the air bubble detector 40 detects air bubbles during priming, the control unit 90G achieves a treatment (air bubble removal treatment) of supplying the transfusion in the transfusion reservoir 60 to the washing liquid reservoir 20 via the transfusion flow path 70, the blood purifier 10, and the washing flow path 30D in order of mention) by causing reverse rotation of the priming pump 80 and activating the transfusion pump 72 (air bubble removal step).

The blood purification device 1G according to the embodiment described above can produce a working effect similar to that of the blood purification device 1 according to the first embodiment.

### <Ninth Embodiment

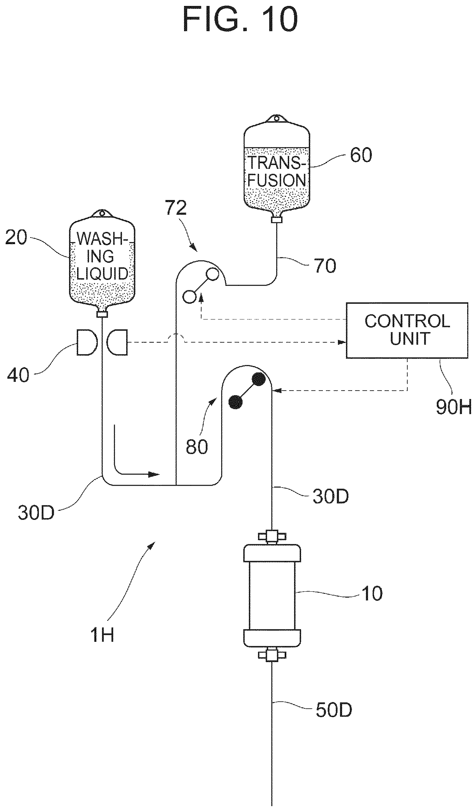

Next, a blood purification device 1H according to the ninth embodiment of the present invention will be described referring to Fig. 10. The blood purification device 1 H according to the present embodiment has a constitution similar to that of the fifth embodiment except the position of the transfusion flow path 70 and the control mode in the fifth embodiment are changed. The components common to those of the fifth embodiment are denoted with the same reference signs as those in the fifth embodiment and a detailed description on these components is omitted.

In the blood purification device 1H according to the present embodiment, the transfusion flow path 70 is connected to the washing flow path 30D on a side upstream of the priming pump 80 thereon as shown in Fig. 10. This means that the priming pump 80 is provided on the washing flow path 30D on a side downstream of the transfusion flow path 70. In the present embodiment, the terms "on a side upstream" and "on a side downstream" are based on a flow direction of a washing liquid at the time of priming and "on the side of the washing liquid reservoir 20" and "on the end-side of the discharge flow path 50D" are called "on a side upstream" and "on a side downstream", respectively.

The control unit 90H in the present embodiment achieves a priming treatment (treatment of delivering a washing liquid in the washing liquid reservoir 20 to the blood purifier 10 via the washing flow path 30D and then discharging the liquid via the discharge flow path 50D) by causing forward rotation of the priming pump 80. When the air bubble detector 40 detects air bubbles during priming, on the other hand, the control unit 90H achieves an air bubble removal treatment (treatment of supplying a transfusion in the transfusion reservoir 60 to the washing liquid reservoir 20 via the transfusion flow path 70 and the washing flow path 30D in order of mention) by stopping the priming pump 80 and activating the transfusion pump 72. The term "forward rotation" of the priming pump 80 means rotation of the priming pump 80 so as to deliver the washing liquid from the upstream side to the downstream side. The term "activating" the transfusion pump 72 means rotating the transfusion pump 72 so as to supply the transfusion from the transfusion reservoir 60 to the washing flow path 30D.

When the air bubble detector 40 detects air bubbles during priming, the control unit 90H in the present embodiment gives an alarm after activating the transfusion pump 72 in a predetermined manner. When the air bubble detector 40 detects air bubbles during priming, the control unit 90H can also give an alarm and stop the transfusion pump 72, and after the alarm is cleared, activate the transfusion pump 72. The control unit 90H can also activate, based on the instruction of an operator, the transfusion pump 72 after the alarm is cleared.

Next, a priming method of the blood purification device 1H according to the present embodiment will be described.

The control unit 90H of the blood purification device 1 H achieves a treatment (priming treatment) of delivering the washing liquid in the washing liquid reservoir 20 to the blood purifier 10 via the washing flow path 30D and then discharging the liquid via the discharge flow path 50D by causing forward rotation of the priming pump 80 (priming step).

Here, when the air bubble detector 40 detects air bubbles during priming, the control unit 90H achieves a treatment (air bubble removal treatment) of supplying the transfusion in the transfusion reservoir 60 to the washing liquid reservoir 20 via the transfusion flow path 70 and the washing flow path 30D in order of mention by stopping the priming pump 80 and activating the transfusion pump 72 (air bubble removal step).

The blood purification device 1H according to the embodiment described above can produce a working effect similar to that of the blood purification device 1 according to the first embodiment.

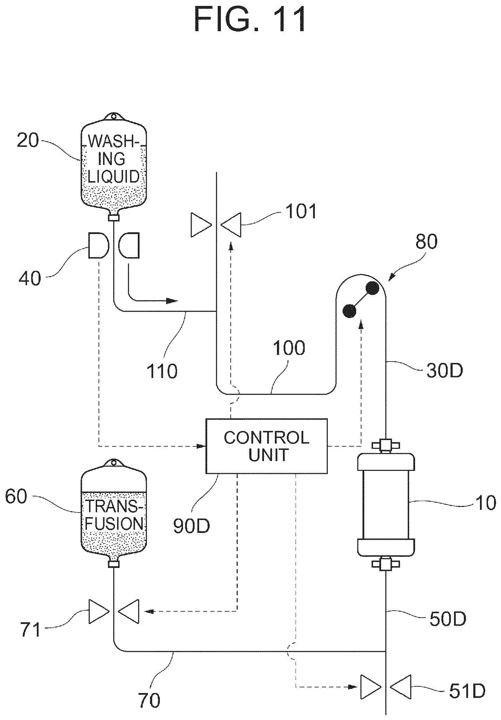

In the fifth to ninth embodiments, shown is an example of using an arterial line that connects a patient's blood removal part and the blood purifier 10 to each other as the washing flow path 30D (Figs. 6 to 10). Alternatively, for example, a priming line 110 connected to an arterial line 100 can be used as a portion of the washing flow path as shown in Fig. 11.

Described specifically, as shown in Fig. 11, it is possible to connect the washing liquid reservoir 20 to the end of the priming line 110 and at the same time, to provide the priming line 110 with the air bubble detector 40. The control unit 90D can achieve a treatment (priming treatment) of delivering a washing liquid in the washing liquid reservoir 20 to the blood purifier 10 via the priming line 110 and the arterial line 100 and then discharging the liquid via the discharge flow path 50D. On the other hand, when the air bubble detector 40 detects air bubbles during priming, the control unit 90D can achieve a treatment (air bubble removal treatment) of supplying a transfusion in the transfusion reservoir 60 to the washing liquid reservoir 20 via the transfusion flow path 70, the discharge flow path 50D, the blood purifier 10, the arterial line 100, and the priming line 110 in order of mention by opening the transfusion flow path 70 by means of the first opening/closing valve 71, closing the discharge flow path 50D by means of the second opening/closing valve 51D, and causing reverse rotation of the priming pump 80. In performing the priming treatment and the air bubble removal treatment, the control unit 90D closes the arterial line 100 by means of a third opening/closing valve 101.

In the above-described embodiments, shown is an example of using an arterial line or venous line as a discharge flow path, but alternatively, another flow path capable of discharging a washing liquid such as filtration line or dialysate line in dialysis can be used as the discharge flow path.

In the above-described embodiments, shown is an example of using physiological saline as the washing liquid or transfusion, but another liquid (dialysate, infusion, fresh frozen plasma, albumin, coelomic fluid, or the like) usable as a washing liquid or transfusion of a blood purifier can also be used. The respective liquids in the washing liquid reservoir 20 and the transfusion reservoir 60 are not necessarily the same and they may be a combination of the above-described liquids (dialysate, infusion, fresh frozen plasma, albumin, coelomic fluid, and the like).

In the above-described embodiments, shown is an application example of the present invention to a blood purification device to be used for leukocyte apheresis (LCAP). The present invention can also be applied to another blood purification device having a similar constitution (blood removal line, blood pump, first transfusion line, transfusion vessel, first opening/closing valve, second transfusion line, second opening/closing valve, transfusion pump). For example, the present invention can also be applied to a blood purification device equipped with a blood purifier for filtration or diffusion to be used for plasma exchange (PE), double filtration plasmapheresis (DFPP), continuous hemofiltration (CHF), continuous hemodialysis (CHD), and continuous hemodiafiltration (CHDF).

### Reference Signs List

1·1A to 1H: Blood purification device
10: Blood purifier
20: Washing liquid reservoir
30-30D: Washing flow path
40: Air bubble detector
50·50D: Discharge flow path
51·51D: Second opening/closing valve
60: Transfusion reservoir
70: Transfusion flow path
71: First opening/closing valve
72: Transfusion pump
80: Priming pump
90·90A to 90H: Control unit
100: Arterial line (washing flow path)
110: Priming line (washing flow path)

## Claims

1. A blood purification device (1), comprising:
a blood purifier (10),
a washing liquid reservoir (20) configured to have a washing liquid stored therein,
a washing flow path (30) configured to connect the blood purifier (10) and the washing liquid reservoir (20) to each other,
a detector (40) provided on the washing flow path (30) configured to detect an air bubble,
a discharge flow path (50) configured to communicate with the blood purifier (10) for discharging the washing liquid,
a transfusion reservoir (60) configured to store a transfusion therein,
a pump (80) provided on a flow path between the detector (40) and the transfusion reservoir (60)**characterized in that** the blood detection device comprises
a control unit (90) configured to activate the pump (80) to supply the transfusion from the transfusion reservoir (60) to the washing liquid reservoir (20) via the detector (40) when the detector (40) is configured to detect an air bubble.

2. The blood purification device (1) according to Claim 1, further comprising:
a transfusion flow path (30) configured to connect the transfusion reservoir (60) and the discharge flow path (50) to each other,
a first opening/closing valve (71) configured to open or close the transfusion flow path (70), and
a second opening/closing valve (51) provided in the discharge flow path on a side downstream of the transfusion flow path (70) configured to open or close the discharge flow path (50), wherein:
the pump (80) is provided on the discharge flow path (50) between the detector (40) and the transfusion flow path (70), and
the control unit (90) configured to at the time of priming, close the transfusion flow path (70) by means of the first opening/closing valve (71), open the discharge flow path (50) by means of the second opening/closing valve (51), and cause forward rotation of the pump (80) and when the detector (40) detects an air bubble, the control unit (90) being configured to supply the transfusion from the transfusion reservoir (60) to the washing liquid reservoir (20) via the detector (40) by opening the transfusion flow path (70) by means of the first opening/closing valve (71), closing the discharge flow path (50) by means of the second opening/closing valve (51), and causing reverse rotation of the pump (80).

3. The blood purification device (1) according to Claim 1, further comprising:
a transfusion flow path (70) configured to connect the transfusion reservoir (60) and the discharge flow path (50) to each other and
a priming pump (80) provided on the discharge flow path (50) on a side downstream of the transfusion flow path (70), wherein:
the pump is a transfusion (72) pump provided on the transfusion flow path (70), and
the control unit (90) being configured to, at the time of priming, cause forward rotation of the priming pump (80) and when the detector (40) detects an air bubble, the control unit (90) is configured to supply the transfusion from the transfusion reservoir (60) to the washing liquid reservoir (20) via the detector (40) by stopping the priming pump (80) and activating the transfusion pump (72).

4. The blood purification device (1) according to Claim 1, further comprising:
a transfusion flow path (70) configured to connect the transfusion reservoir (60) and the discharge flow path (50) to each other and
a transfusion pump (72) provided on the transfusion flow path (70), wherein:
the pump is a priming pump (80) provided on the discharge flow path (50) on a side upstream of the transfusion flow path (70), and
the control unit (90) configured to, at the time of priming, cause forward rotation of the priming pump (80) and when the detector (40) detects an air bubble, the control unit (90) being configured to supply the transfusion from the transfusion reservoir (60) to the washing liquid reservoir (20) via the detector(40) by causing reverse rotation of the priming pump (80) and activating the transfusion pump (72).

5. The blood purification device (1) according to Claim 4, wherein in causing reverse rotation of the priming pump (80) and activating the transfusion pump (72), the control unit (90) controls a flow rate of the priming pump (80) to be smaller than a flow rate of the transfusion pump (72).

6. The blood purification device (1) according to Claim 1, further comprising:
a transfusion flow path (70) configured to connect the transfusion reservoir (60) and the washing flow path (30) or the blood purifier (10) to each other, and
a priming pump (80) provided on the discharge flow path (50), wherein:
the pump is a transfusion pump (72) provided on the transfusion flow path (70) and
the control unit (90) configured to, at the time of priming, cause forward rotation of the priming pump (80) and when the detector (40) detects an air bubble, the control unit (90) being configured to supply the transfusion from the transfusion reservoir (60) to the washing liquid reservoir (20) via the detector (40) by stopping the priming pump (80) and activating the transfusion pump (72).

7. The blood purification device (1) according to any one of Claims 2 to 6, wherein the washing flow path (30) is a venous line that connects a patient's blood return part and the blood purifier (10) to each other.

8. The blood purification device (1) according to Claim 1, further comprising:
a transfusion flow path (70) configured to connect the transfusion reservoir (60) and the discharge flow path (50) to each other,
a first opening/closing valve (71) configured to open or close the transfusion flow path (70), and
a second opening/closing valve (51) provided in the discharge flow path (50) on a side downstream of the transfusion flow path (70), wherein:
the pump (80) is provided on the washing flow path (30), and
the control unit (90) configured to, at the time of priming, close the transfusion flow path (70) by means of the first opening/closing valve (71), opens the discharge flow path (50) by means of the second opening/closing valve (51), and causes forward rotation of the pump (80) and when the detector (40) detects an air bubble, the control unit (90) being configured to supply the transfusion from the transfusion reservoir (60) to the washing liquid reservoir (20) via the detector (40) by opening the transfusion flow path (70) by means of the first opening/closing valve (71), closing the discharge flow path (50) by means of the second opening/closing valve (51), and causing reverse rotation of the pump (80).

9. The blood purification device (1) according to Claim 1, further comprising:
a transfusion flow path (70) configured to connect the transfusion reservoir (60) and the discharge flow path (50) to each other, and
a priming pump (80) provided on the washing flow path (30), wherein:
the pump is a transfusion pump (72) provided on the transfusion flow path (70), and
the control unit (90) being configured to, at the time of priming, cause forward rotation of the priming pump (80) and when the detector (40) detects an air bubble, the control unit (90) being configured to supply the transfusion from the transfusion reservoir (60) to the washing liquid reservoir (20) via the detector (40) by causing reverse rotation of the priming pump (80) and activating the transfusion pump (72).

10. The blood purification device (1) according to Claim 1, further comprising:
a transfusion flow path (70) configured to connect the transfusion reservoir (60) and the washing flow path (30) to each other, and
a priming pump (80) provided on the washing flow path (30) on a side upstream of the transfusion flow path (30), wherein:
the pump is a transfusion pump (72) provided on the transfusion flow path (70), and
the control unit (90) being configured to, at the time of priming, cause forward rotation of the priming pump (80) and when the detector (40) detects an air bubble, the control unit (90) being configured to supply the transfusion from the transfusion reservoir (60) to the washing liquid reservoir (20) via the detector (40) by causing reverse rotation of the priming pump (80) and activating the transfusion pump (72).

11. The blood purification device (1) according to Claim 1, further comprising:
a transfusion flow path (70) configured to connect the transfusion reservoir (60) and the blood purifier (10) to each other, and
a priming pump (80) provided on the washing flow path (30), wherein:
the pump is a transfusion pump (72) provided on the transfusion flow path (70), and
the control unit (90) being configured to, at the time of priming, cause forward rotation of the priming pump (80) and when the detector (40) detects an air bubble, the control unit (90) being configured to supply the transfusion from the transfusion reservoir (60) to the washing liquid reservoir (20) via the detector (40) by causing reverse rotation of the priming pump (80) and activating the transfusion pump (72).

12. The blood purification device (1) according to any one of Claims 9 to 11, wherein in causing reverse rotation of the priming pump (80) and activating the transfusion pump (72), the control unit (90) being configured to control a flow rate of the priming pump (80) to be smaller than a flow rate of the transfusion pump (72).

13. The blood purification device (1) according to Claim 1, further comprising:
a transfusion flow path (70) configured to connect the transfusion reservoir (60) and the washing flow path (30) to each other, and
a priming pump (80) provided on the washing flow path (30) on a side downstream of the transfusion flow path (70), wherein:
the pump is a transfusion pump (72) provided on the transfusion flow path (70), and
the control unit (90) being configured to, at the time of priming, cause forward rotation of the priming pump (80) and when the detector (40) detects an air bubble, the control unit (90) being configured to supply the transfusion from the transfusion reservoir (60) to the washing liquid reservoir (20) via the detector (40) by stopping the priming pump (80) and activating the transfusion pump (72).

14. The blood purification device (1) according to any one of Claims 8 to 13, wherein the washing flow path (30) is an arterial line configured to connect a patient's blood removal part and the blood purifier (10) to each other.

15. The blood purification device (1) according to any one of Claims 8 to 13, wherein the washing flow path (30) has a priming line placed in an arterial line configured to connect a patient's blood removal part and the blood purifier (10) to each other.

16. The blood purification device (1) according to any one of Claims 1 to 15, wherein when the detector (40) detect an air bubble, the control unit (90) is configured togive an alarm after activating the pump (80) in a predetermined manner.

17. The blood purification device (1) according to any one of Claims 1 to 15, wherein when the detector (40) detect an air bubble, the control unit (90) is configured to give an alarm and stops the pump and after the alarm is cleared, activates the pump (80).

18. A method of priming a blood purification device (1) equipped with: a blood purifier (10); a washing liquid reservoir (20) configured to store a washing liquid therein; a washing flow path (30) configured to connect the blood purifier (10) and the washing liquid reservoir (20) to each other; a detector (40) provided on the washing flow path (30) for detecting an air bubble; a discharge flow path (50) configured to communicate with the blood purifier (10) for discharging the washing liquid; a transfusion reservoir (60) configured to store a transfusion therein; and a pump (80) provided on a flow path (40) between the detector and the transfusion reservoir (60),
the method comprising a step of, when the detector (40) detects an air bubble during priming, activating the pump (80) so as to supply the transfusion from the transfusion reservoir (60) to the washing liquid reservoir (20) via the detector (40).

## Patentansprüche

1. Blutreinigungsvorrichtung (1), umfassend:
einen Blutreiniger (10);
ein Waschflüssigkeitsreservoir (20), das so konfiguriert ist, dass Waschflüssigkeit darin aufbewahrt werden kann;
einen Waschfließweg (30), der so konfiguriert ist, dass er den Blutreiniger (10) und das Waschflüssigkeitsreservoir (20) miteinander verbindet;
einen Detektor (40), der sich auf dem Waschfließweg (30) befindet und der so konfiguriert ist, dass er eine Luftblase nachweisen kann;
einen Austragsfließweg (50), der so konfiguriert ist, dass er mit dem Blutreiniger (10) kommunizieren kann, um die Waschflüssigkeit auszutragen;
ein Transfusionsreservoir (60), das so konfiguriert ist, dass eine Transfusionsflüssigkeit darin aufbewahrt werden kann;
eine Pumpe (80), die sich auf einem Fließweg zwischen dem Detektor (40) und dem Transfusionsreservoir (60) befindet;
**dadurch gekennzeichnet, dass** die Blutnachweisvorrichtung eine Steuerungseinheit (90) umfasst, die so konfiguriert ist, dass sie die Pumpe (80) zum Zuführen der Transfusionsflüssigkeit aus dem Transfusionsreservoir (60) über den Detektor (40) zum Waschflüssigkeitsreservoir (20) aktivieren kann, wenn der Detektor (40) so konfiguriert ist, dass er eine Luftblase nachweisen kann.

2. Blutreinigungsvorrichtung (1) gemäß Anspruch 1, weiterhin umfassend:
einen Transfusionsfließweg (30), der so konfiguriert ist, dass er das Transfusionsreservoir (60) und den Austragsfließweg (50) miteinander verbindet;
ein erstes Öffnungs-/Schließ-Ventil (71), das so konfiguriert ist, dass es den Transfusionsfließweg (70) öffnen oder schließen kann; und
ein zweites Öffnungs-/Schließ-Ventil (51), das sich im Austragsfließweg auf einer Seite stromabwärts des Transfusionsfließwegs (70) befindet, wobei:
sich die Pumpe (80) auf dem Austragsfließweg (50) zwischen dem Detektor (40) und dem Transfusionsfließweg (70) befindet; und
die Steuerungseinheit (90) so konfiguriert ist, dass sie zum Zeitpunkt der Vorbereitung den Transfusionsfließweg (70) mittels des ersten Öffnungs-/ Schließ-Ventils (71) schließt, den Austragsfließweg (50) mittels des zweiten Öffnungs-/Schließ-Ventils (51) öffnet und eine Vorwärtsrotation der Pumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie den Transfusionsfließweg (70) mittels des ersten Öffnungs-/Schließ-Ventils (71) öffnet, den Austragsfließweg (50) mittels des zweiten Öffnungs-/ Schließ-Ventils (51) schließt und eine umgekehrte Rotation der Pumpe (80) bewirkt.

3. Blutreinigungsvorrichtung (1) gemäß Anspruch 1, weiterhin umfassend:
einen Transfusionsfließweg (70), der so konfiguriert ist, dass er das Transfusionsreservoir (60) und den Austragsfließweg (50) miteinander verbindet; und
eine Vorbereitungspumpe (80), die sich auf dem Austragsfließweg (50) auf einer Seite stromabwärts des Transfusionsfließwegs (70) befindet, wobei:
die Pumpe eine Transfusionspumpe (72) ist, die sich auf dem Transfusionsfließweg (70) befindet; und
die Steuerungseinheit (90) so konfiguriert ist, dass sie zum Zeitpunkt der Vorbereitung eine Vorwärtsrotation der Vorbereitungspumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie die Vorbereitungspumpe (80) ausschaltet und die Transfusionspumpe (72) aktiviert.

4. Blutreinigungsvorrichtung (1) gemäß Anspruch 1, weiterhin umfassend:
einen Transfusionsfließweg (70), der so konfiguriert ist, dass er das Transfusionsreservoir (60) und den Austragsfließweg (50) miteinander verbindet; und
eine Transfusionspumpe (72), die sich auf dem Transfusionsfließweg (70) befindet, wobei:
die Pumpe eine Vorbereitungspumpe (80) ist, die sich auf dem Austragsfließweg (50) auf einer Seite stromaufwärts des Transfusionsfließwegs (70) befindet, und
die Steuerungseinheit (90) so konfiguriert ist, dass sie zum Zeitpunkt der Vorbereitung eine Vorwärtsrotation der Vorbereitungspumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie eine umgekehrte Rotation der Vorbereitungspumpe (80) bewirkt und die Transfusionspumpe (72) aktiviert.

5. Blutreinigungsvorrichtung (1) gemäß Anspruch 4, wobei die Steuerungseinheit (90), indem sie eine umgekehrte Rotation der Vorbereitungspumpe (80) bewirkt und die Transfusionspumpe (72) aktiviert, die Fließgeschwindigkeit der Vorbereitungspumpe (80) so steuert, dass sie geringer ist als die Fließgeschwindigkeit der Transfusionspumpe (72).

6. Blutreinigungsvorrichtung (1) gemäß Anspruch 1, weiterhin umfassend:
einen Transfusionsfließweg (70), der so konfiguriert ist, dass er das Transfusionsreservoir (60) und den Waschfließweg (30) oder den Blutreiniger (10) miteinander verbindet; und
eine Vorbereitungspumpe (80), die sich auf dem Austragsfließweg (50) befindet, wobei:
die Pumpe eine Transfusionspumpe (72) ist, die sich auf dem Transfusionsfließweg (70) befindet; und
die Steuerungseinheit (90) so konfiguriert ist, dass sie zum Zeitpunkt der Vorbereitung eine Vorwärtsrotation der Vorbereitungspumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie die Vorbereitungspumpe (80) ausschaltet und die Transfusionspumpe (72) aktiviert.

7. Blutreinigungsvorrichtung (1) gemäß einem der Ansprüche 2 bis 6, wobei der Waschfließweg (30) eine venöse Leitung ist, die das Blutrückführungsteil eines Patienten und den Blutreiniger (10) miteinander verbindet.

8. Blutreinigungsvorrichtung (1) gemäß Anspruch 1, weiterhin umfassend:
einen Transfusionsfließweg (70), der so konfiguriert ist, dass er das Transfusionsreservoir (60) und den Austragsfließweg (50) miteinander verbindet;
ein erstes Öffnungs-/Schließ-Ventil (71), das so konfiguriert ist, dass es den Transfusionsfließweg (70) öffnen oder schließen kann; und
ein zweites Öffnungs-/Schließ-Ventil (51), das sich im Austragsfließweg (50) auf einer Seite stromabwärts des Transfusionsfließwegs (70) befindet, wobei:
sich die Pumpe (80) auf dem Waschfließweg (30) befindet; und
die Steuerungseinheit (90) so konfiguriert ist, dass sie zum Zeitpunkt der Vorbereitung den Transfusionsfließweg (70) mittels des ersten Öffnungs-/ Schließ-Ventils (71) schließt, den Austragsfließweg (50) mittels des zweiten Öffnungs-/Schließ-Ventils (51) öffnet und eine Vorwärtsrotation der Pumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie den Transfusionsfließweg (70) mittels des ersten Öffnungs-/Schließ-Ventils (71) öffnet, den Austragsfließweg (50) mittels des zweiten Öffnungs-/ Schließ-Ventils (51) schließt und eine umgekehrte Rotation der Pumpe (80) bewirkt.

9. Blutreinigungsvorrichtung (1) gemäß Anspruch 1, weiterhin umfassend:
einen Transfusionsfließweg (70), der so konfiguriert ist, dass er das Transfusionsreservoir (60) und den Austragsfließweg (50) miteinander verbindet; und
eine Vorbereitungspumpe (80), die sich auf dem Waschfließweg (30) befindet; wobei
die Pumpe eine Transfusionspumpe (72) ist, die sich auf dem Transfusionsfließweg (70) befindet; und
die Steuerungseinheit (90) so konfiguriert ist, dass sie zum Zeitpunkt der Vorbereitung eine Vorwärtsrotation der Vorbereitungspumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie eine umgekehrte Rotation der Vorbereitungspumpe (80) bewirkt und die Transfusionspumpe (72) aktiviert.

10. Blutreinigungsvorrichtung (1) gemäß Anspruch 1, weiterhin umfassend:
einen Transfusionsfließweg (70), der so konfiguriert ist, dass er das Transfusionsreservoir (60) und den Waschfließweg (30) miteinander verbindet; und
eine Vorbereitungspumpe (80), die sich auf dem Waschfließweg (30) auf einer Seite stromaufwärts des Transfusionsfließwegs (30) befindet, wobei:
die Pumpe eine Transfusionspumpe (72) ist, die sich auf dem Transfusionsfließweg (70) befindet; und
die Steuerungseinheit (90) so konfiguriert ist, dass sie zum Zeitpunkt der Vorbereitung eine Vorwärtsrotation der Vorbereitungspumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie eine umgekehrte Rotation der Vorbereitungspumpe (80) bewirkt und die Transfusionspumpe (72) aktiviert.

11. Blutreinigungsvorrichtung (1) gemäß Anspruch 1, weiterhin umfassend:
einen Transfusionsfließweg (70), der so konfiguriert ist, dass er das Transfusionsreservoir (60) und den Blutreiniger (10) miteinander verbindet; und
eine Vorbereitungspumpe (80), die sich auf dem Waschfließweg (30) befindet, wobei:
die Pumpe eine Transfusionspumpe (72) ist, die sich auf dem Transfusionsfließweg (70) befindet; und
die Steuerungseinheit (90) so konfiguriert ist, dass sie zum Zeitpunkt der Vorbereitung eine Vorwärtsrotation der Vorbereitungspumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie eine umgekehrte Rotation der Vorbereitungspumpe (80) bewirkt und die Transfusionspumpe (72) aktiviert.

12. Blutreinigungsvorrichtung (1) gemäß einem der Ansprüche 9 bis 11, wobei die Steuerungseinheit (90), indem sie eine umgekehrte Rotation der Vorbereitungspumpe (80) bewirkt und die Transfusionspumpe (72) aktiviert, die Fließgeschwindigkeit der Vorbereitungspumpe (80) so steuern kann, dass sie geringer ist als die Fließgeschwindigkeit der Transfusionspumpe (72).

13. Blutreinigungsvorrichtung (1) weiterhin umfassend:
einen Transfusionsfließweg (70), der so konfiguriert ist, dass er das Transfusionsreservoir (60) und den Waschfließweg (30) miteinander verbindet; und
eine Vorbereitungspumpe (80), die sich auf dem Waschfließweg (30) auf einer Seite stromabwärts des Transfusionsfließwegs (70) befindet, wobei:
die Pumpe eine Transfusionspumpe (72) ist, die sich auf dem Transfusionsfließweg (70) befindet; und
die Steuerungseinheit (90) so konfiguriert ist, dass sie zum Zeitpunkt der Vorbereitung eine Vorwärtsrotation der Vorbereitungspumpe (80) bewirkt, und wenn der Detektor (40) eine Luftblase nachweist, die Steuerungseinheit (90) so konfiguriert ist, dass sie die Transfusionsflüssigkeit aus dem Transfusionsreservoir (60) über den Detektor (40) zum Waschflüssigkeitsreservoir (20) zuführen kann, indem sie die Vorbereitungspumpe (80) ausschaltet und die Transfusionspumpe (72) aktiviert.

14. Blutreinigungsvorrichtung (1) gemäß einem der Ansprüche 8 bis 13, wobei der Waschfließweg (30) eine arterielle Leitung ist, die so konfiguriert ist, dass sie das Blutentnahmeteil eines Patienten und den Blutreiniger (10) miteinander verbinden kann.

15. Blutreinigungsvorrichtung (1) gemäß einem der Ansprüche 8 bis 13, wobei der Waschfließweg (30) eine Vorbereitungsleitung aufweist, die in einer arteriellen Leitung platziert ist, welche so konfiguriert ist, dass sie das Blutentnahmeteil eines Patienten und den Blutreiniger (10) miteinander verbinden kann.

16. Blutreinigungsvorrichtung (1) gemäß einem der Ansprüche 1 bis 15, wobei dann, wenn der Detektor (40) eine Luftblase nachweist, die Steuerungseinheit (90) so konfiguriert ist, dass sie einen Alarm auslöst, nachdem sie die Pumpe (80) in einer vorbestimmten Weise aktiviert hat.

17. Blutreinigungsvorrichtung (1) gemäß einem der Ansprüche 1 bis 15, wobei dann, wenn der Detektor (40) eine Luftblase nachweist, die Steuerungseinheit (90) so konfiguriert ist, dass sie einen Alarm auslöst und die Pumpe ausschaltet und, nachdem der Alarm quittiert ist, die Pumpe (80) aktiviert.

18. Verfahren zum Vorbereiten einer Blutreinigungsvorrichtung (1), die ausgestattet ist mit: einem Blutreiniger (10); einem Waschflüssigkeitsreservoir (20), das so konfiguriert ist, dass Waschflüssigkeit darin aufbewahrt werden kann; einem Waschfließweg (30), der so konfiguriert ist, dass er den Blutreiniger (10) und das Waschflüssigkeitsreservoir (20) miteinander verbindet; einem Detektor (40), der sich auf dem Waschfließweg (30) befindet, um eine Luftblase nachzuweisen; einem Austragsfließweg (50), der so konfiguriert ist, dass er mit dem Blutreiniger (10) kommunizieren kann, um die Waschflüssigkeit auszutragen; einem Transfusionsreservoir (60), das so konfiguriert ist, dass eine Transfusionsflüssigkeit darin aufbewahrt werden kann; und einer Pumpe (80), die sich auf einem Fließweg (40) zwischen dem Detektor und dem Transfusionsreservoir (60) befindet;
wobei das Verfahren einen Schritt umfasst, bei dem, wenn der Detektor (40) während der Vorbereitung eine Luftblase nachweist, die Pumpe (80) aktiviert wird, so dass die Transfusionsflüssigkeit aus dem Transfusionsreservoir (60) über den Detektor (40) zum Waschflüssigkeitsreservoir (20) zugeführt wird.

## Revendications

1. Dispositif (1) de purification du sang, comprenant :
un purificateur de sang (10),
un réservoir (20) de liquide de lavage configuré pour stocker un liquide de lavage,
une tubulure de lavage (30) configurée pour relier l'un à l'autre le purificateur de sang (10) et le réservoir (20) de liquide de lavage,
un détecteur (40), disposé sur la tubulure de lavage (30), configuré pour détecter une bulle d'air,
une tubulure d'évacuation (50), configurée pour communiquer avec le purificateur de sang (10) pour évacuer le liquide de lavage,
un réservoir de transfusion (60), configuré pour stocker une transfusion,
une pompe (80) disposée sur une tubulure entre le détecteur (40) et le réservoir de transfusion (60), **caractérisé en ce que** le dispositif de détection du sang comprend
une unité de commande (90) configurée pour activer la pompe (80) de façon à envoyer la transfusion du réservoir de transfusion (60) vers le réservoir (20) de liquide de lavage en passant par le détecteur (40) lorsque le détecteur (40) est configuré pour détecter une bulle d'air.

2. Dispositif (1) de purification du sang selon la revendication 1, comprenant en outre :
une tubulure de transfusion (30) configurée de façon à relier l'un à l'autre le réservoir de transfusion (60) et la tubulure d'évacuation (50),
une première vanne d'ouverture/fermeture (71) configurée pour ouvrir ou fermer la tubulure de transfusion (70), et
une seconde vanne d'ouverture/fermeture (51) disposée dans la tubulure d'évacuation sur un côté aval de la tubulure de transfusion (70), et configurée pour ouvrir ou fermer la tubulure d'évacuation (50), dans lequel
la pompe (80) est disposée sur la tubulure d'évacuation (50) entre le détecteur (40) et la tubulure de transfusion (70), et
l'unité de commande (90) est, au moment de l'amorçage, configurée pour fermer la tubulure de transfusion (70) au moyen de la première vanne d'ouverture/fermeture (71), ouvrir la tubulure d'évacuation (50) au moyen de la seconde vanne d'ouverture/fermeture (51) et provoquer une rotation avant de la pompe (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40) en ouvrant la tubulure de transfusion (70) au moyen de la première vanne d'ouverture/fermeture (71), en fermant la tubulure d'évacuation (50) au moyen de la seconde vanne d'ouverture/fermeture (51), et en provoquant une rotation inversée de la pompe (80).

3. Dispositif (1) de purification du sang selon la revendication 1, comprenant en outre :
une tubulure de transfusion (70) configurée de façon à relier l'un à l'autre le réservoir de transfusion (60) et la tubulure d'évacuation (50) et
une pompe d'amorçage (80) disposée sur la tubulure d'évacuation (50) sur un côté aval de la tubulure de transfusion (70), dans lequel :
la pompe est une pompe de transfusion (72) disposée sur la tubulure de transfusion (70), et
l'unité de commande (90) étant configurée de façon à provoquer, au moment de l'amorçage, une rotation avant de la pompe d'amorçage (80), et lorsque le détecteur (40) détecte une bulle d'air, l'unité de commande (90) est configurée pour envoyer la transfusion du réservoir de transfusion (60) vers le réservoir (20) de liquide de lavage en passant par le détecteur (40), en arrêtant la pompe d'amorçage (80) et en activant de la pompe de transfusion (72).

4. Dispositif (1) de purification du sang selon la revendication 1, comprenant en outre :
une tubulure de transfusion (70) configurée pour relier l'un à l'autre le réservoir de transfusion (60) et la tubulure d'évacuation (50), et
une pompe de transfusion (72) disposée sur la tubulure de transfusion (70), dans lequel :
la pompe est une pompe d'amorçage (80) disposée sur la tubulure d'évacuation (50) sur un côté amont de la tubulure de transfusion (70), et
l'unité de commande (90) étant configurée de façon à provoquer, au moment de l'amorçage, une rotation avant de la pompe d'amorçage (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40) en provoquant une rotation inversée de la pompe d'amorçage (80) et en activant la pompe de transfusion (72).

5. Dispositif (1) de purification du sang selon la revendication 4, dans lequel, en provoquant une rotation inversée de la pompe d'amorçage (80) et en activant la pompe de transfusion (72), l'unité de commande (90) commande un débit de la pompe d'amorçage (80) qui est inférieur à un débit de la pompe de transfusion (72).

6. Dispositif (1) de purification du sang selon la revendication 1, comprenant en outre :
une tubulure de transfusion (70) configurée pour relier l'un à l'autre le réservoir de transfusion (60) et la tubulure de lavage (30) ou le purificateur de sang (10), et
une pompe d'amorçage (80) disposée sur la tubulure d'évacuation (50), dans lequel :
la pompe est une pompe de transfusion (72) disposée sur la tubulure de transfusion (70) et
l'unité de commande (90) étant configurée de façon à provoquer, au moment de l'amorçage, une rotation avant de la pompe d'amorçage (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40) en arrêtant la pompe d'amorçage (80) et en activant la pompe de transfusion (72).

7. Dispositif (1) de purification du sang selon l'une quelconque des revendications 2 à 6, dans lequel la tubulure de lavage (30) est une ligne veineuse qui relie l'un à l'autre une partie de retour du sang du patient et le purificateur de sang (10).

8. Dispositif (1) de purification du sang selon la revendication 1, comprenant en outre :
une tubulure de transfusion (70) configurée pour relier l'un à l'autre le réservoir de transfusion (60) et la tubulure d'évacuation (50),
une première vanne d'ouverture/fermeture (71) configurée pour ouvrir ou fermer la tubulure de transfusion (70), et
une seconde vanne d'ouverture/fermeture (51) disposée sur la tubulure d'évacuation (50) sur un côté aval de la tubulure de transfusion (70), dans lequel :
la pompe (80) est disposée sur la tubulure de lavage (30), et
l'unité de commande (90) étant configurée de façon à fermer, au moment de l'amorçage, la tubulure de transfusion (70) au moyen de la première vanne d'ouverture/fermeture (71), ouvrir la tubulure d'évacuation (50) au moyen de la seconde vanne d'ouverture/fermeture (51), et provoquer une rotation avant de la pompe (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40) en ouvrant la tubulure de transfusion (70) au moyen de la première vanne d'ouverture/fermeture (71), en fermant la tubulure d'évacuation (50) au moyen de la seconde vanne d'ouverture/fermeture (51), et en provoquant une rotation inversée de la pompe (80).

9. Dispositif (1) de purification du sang selon la revendication 1, comprenant en outre :
une tubulure de transfusion (70) configurée pour relier l'un à l'autre le réservoir de transfusion (60) et la tubulure d'évacuation (50), et
une pompe d'amorçage (80) disposée sur la tubulure de lavage (30), dans lequel :
la pompe est une pompe de transfusion (72) disposée sur la tubulure de transfusion (70), et
l'unité de commande (90) étant configurée de façon à provoquer, au moment de l'amorçage, une rotation avant de la pompe d'amorçage (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40), en provoquant la rotation inversée de la pompe d'amorçage (80) et en activant la pompe de transfusion (72).

10. Dispositif (1) de purification du sang selon la revendication 1, comprenant en outre :
une tubulure de transfusion (70) configurée pour relier l'un à l'autre le réservoir de transfusion (60) et la tubulure de lavage (30), et
une pompe d'amorçage (80) disposée sur la tubulure de lavage (30) sur un côté amont de la tubulure de transfusion (30), dans lequel :
la pompe est une pompe de transfusion (72) disposée sur la tubulure de transfusion (70), et
l'unité de commande (90) étant configurée de façon à provoquer, au moment de l'amorçage, une rotation avant de la pompe d'amorçage (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40), en provoquant une rotation inversée de la pompe d'amorçage (80) et en activant la pompe de transfusion (72).

11. Dispositif (1) de purification du sang selon la revendication 1, comprenant en outre :
une tubulure de transfusion (70) configurée pour relier l'un à l'autre le réservoir de transfusion (60) et le purificateur de sang (10), et
une pompe d'amorçage (80) disposée sur la tubulure de lavage (30), dans lequel :
la pompe est une pompe de transfusion (72) disposée sur la tubulure de transfusion (70), et
l'unité de commande (90) étant configurée de façon à provoquer, au moment de l'amorçage, une rotation avant de la pompe d'amorçage (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40), en provoquant une rotation inversée de la pompe d'amorçage (80) et en activant la pompe de transfusion (72).

12. Dispositif (1) de purification du sang selon l'une quelconque des revendications 9 à 11, dans lequel, en provoquant une rotation inversée de la pompe d'amorçage (80) et en activant la pompe de transfusion (72), l'unité de commande (90) est configurée pour commander un débit de la pompe d'amorçage (80) qui est inférieur à un débit de la pompe de transfusion (72).

13. Dispositif (1) de purification du sang selon la revendication 1, comprenant en outre :
une tubulure de transfusion (70) configurée pour relier l'un à l'autre le réservoir de transfusion (60) et la tubulure de lavage (30), et
une pompe d'amorçage (80) disposée sur la tubulure de lavage (30) sur un côté aval de la tubulure de transfusion (70), dans lequel :
la pompe est une pompe de transfusion (72) disposée sur la tubulure de transfusion (70), et
l'unité de commande (90) étant configurée de façon à provoquer, au moment de l'amorçage, une rotation avant de la pompe d'amorçage (80), et lorsque le détecteur (40) détecte une bulle d'air, l'unité de commande (90) étant configurée pour envoyer la transfusion du réservoir de transfusion (60) vers le réservoir (20) de liquide de lavage en passant par le détecteur (40), en arrêtant la pompe d'amorçage (80) et en activant la pompe de transfusion (72).

14. Dispositif (1) de purification du sang selon l'une quelconque des revendications 8 à 13, dans lequel la tubulure de lavage (30) est une ligne artérielle configurée pour relier l'un à l'autre une partie d'évacuation du sang du patient et le purificateur de sang (10).

15. Dispositif (1) de purification du sang selon l'une quelconque des revendications 8 à 13, dans lequel la tubulure de lavage (30) possède une ligne d'amorçage placée dans une ligne artérielle configurée pour relier l'un à l'autre une partie d'évacuation du sang du patient et le purificateur de sang (10).

16. Dispositif (1) de purification du sang selon l'une quelconque des revendications 1 à 15, dans lequel, lorsque le détecteur (40) détecte une bulle d'air, l'unité de commande (90) est configurée pour déclencher une alarme après activation de la pompe (80) d'une manière prédéterminée.

17. Dispositif (1) de purification du sang selon l'une quelconque des revendications 1 à 15, dans lequel, lorsque le détecteur (40) détecte une bulle d'air, l'unité de commande (90) est configurée pour déclencher une alarme et arrête la pompe et, après acquittement de l'alarme, active la pompe (80).

18. Procédé d'amorçage d'un dispositif (1) de purification du sang, équipé de : un purificateur de sang (10) ; un réservoir (20) de liquide de lavage configuré pour stocker un liquide de lavage ; une tubulure de lavage (30) configurée pour relier l'un à l'autre le purificateur de sang (10) et le réservoir (20) de liquide de lavage ; un détecteur (40) disposé sur la tubulure de lavage (30) pour détecter une bulle d'air ; une tubulure d'évacuation (50) configurée pour communiquer avec le purificateur de sang (10) pour évacuer le liquide de lavage ; un réservoir de transfusion (60) configuré pour stocker une transfusion ; et une pompe (80) disposée sur une tubulure (40) entre le détecteur et le réservoir de transfusion (60),
le procédé comprenant une étape consistant à, lorsque le détecteur (40) détecte une bulle d'air pendant l'amorçage, activer la pompe (80) de façon à envoyer la transfusion du réservoir de transfusion (60) vers le réservoir (20) de liquide de lavage en passant par le détecteur (40).
